# EUROPÄISCHE PATENTSCHRIFT

(11) **EP 2 367 498 B1**
(45) Veröffentlichungstag und Bekanntmachung des Hinweises auf die Patenterteilung: **03.07.2013**
(21) Anmeldenummer: 09744022.6
(22) Anmeldetag: 20.10.2009
(51) Int. Cl.: A61C 1/08, A61C 1/00, A61B 19/00, A61B 17/00, A61B 17/17, A61B 17/16, A61B 17/88

(54) **FRÄSEINRICHTUNG ZUM EINBRINGEN EINER KNOCHENBOHRUNG**
DRILLING APPARATUS FOR A BONE BORE
FRAISE POUR MORTAISER L'OS

(30) Priorität: 28.10.2008 AT 16802008; 10.12.2008 AT 19232008
(43) Veröffentlichungstag der Anmeldung: 28.09.2011
(73) Patentinhaber: JEDER GMBH, 1190 Wien (AT)
(72) Erfinder: EDER, Klaus, A-2380 Perchtoldsdorf (AT)
(74) Vertreter: Wildhack & Jellinek
(86) Internationale Anmeldenummer: PCT/AT2009/000410
(87) Internationale Veröffentlichungsnummer: WO 2010/048648

(56) Entgegenhaltungen:
- EP-A2- 1 269 933
- WO-A1-2007/129312
- WO-A1-2009/000052
- DE-A1-102007 059 226
- US-B1- 6 358 252

## Beschreibung

Die Erfindung betrifft eine Einrichtung zum durchdringenden Verlängern einer in hartes Gewebe, insbesondere den Kieferknochen, eingebrachten Sackbohrung.

Eine solche Verlängerung einer Knochenbohrung ist beispielsweise im Bereich der Zahnchirurgie bei der Durchführung eines als "Sinuslift" bezeichneten Eingriffs erforderlich. Als Sinuslift wird eine Operation bezeichnet, bei der die Kieferhöhlenschleimhaut (Sinushaut) vom Kieferknochen teilweise abgelöst und angehoben wird, um einen Raum zwischen Knochen und Kieferhöhlenschleimhaut herzustellen. In den entstandenen Hohlraum wird nun autologer Knochen (z.B. vom Tuber maxillae, der Linea obliqua, der Kinnregion oder aus dem Beckenkamm (Knochenersatzmaterialien, Knochenspan)) bzw. ein synthetisches Knochersatzmaterial (zum Beispiel Knochenersatzmaterial der Marke Bio-Oss der Firma Geistlich AG) häufig vermischt mit autologem Knochen, eingebracht. Dieses Material soll sich innerhalb von 6 Monaten zu Knochen umbauen, um ein solides Fundament für ein Implantat zu gewährleisten.

Die herkömmliche Vorgehensweise bei einem Sinuslift ist es, im Molarenbereich buccal einen Mucoperiostlappen zu präparieren, und in den dadurch freigelegten Knochen ein ovales Fenster zu fräsen, ohne die darunterliegende Kieferhöhlenschleimhaut zu beschädigen. Die an der Kieferhöhlenschleimhaut hängende, ovale Knochenscheibe wird nun vorsichtig in Richtung der Kieferhöhle gedrückt, wobei gleichzeitig die Kieferhöhlenschleimhaut rund um das Fenster vorsichtig mit speziellen Instrumenten vom Knochen abgelöst wird. Da die Kieferhöhlenschleimhaut sehr zart ist, etwa vergleichbar mit einer Eihaut, ist dieser Vorgang sehr vorsichtig auszuführen, da die Gefahr besteht, die Kieferhöhlenschleimhaut zu beschädigen. Der so in der Kieferhöhle entstandene Raum wird nun durch das Fenster mit dem Knochenersatzmaterial aufgefüllt und das buccale Fenster wird mit einer Folie abgedeckt. Die Folie besteht im Allgemeinen aus einem resorbierbaren Material, wie etwa einer Membran der Marke Bio-Gide der Firma Geistlich AG. Danach wird der Mucoperiostlappen dicht vernäht. Die Methode ist verhältnismäßig stark invasiv und belastet den Patienten durch starke Schwellung und Verfärbung bis zu 10 Tage, eventuell auch durch Schmerzen.

Dieses Operationsverfahren wird oft auch als "offener" oder "klassischer" Sinuslift bezeichnet. Falls eine ausreichende Restknochenhöhe vorhanden ist (etwa mit einer Höhe von 5mm), können die Implantate "zeitgleich mit dem Sinuslift eingesetzt werden (einzeitiger Sinuslift). Eine volle Belastung der Implantate ist erst nach der Verfestigung des Knochenersatzmaterials möglich. Wenn die Restknochenhöhe zu dünn ist, erfolgt das Einsetzen der Implantate in einem zweiten Eingriff etwa 6-8 Monate nach dem Sinuslift (zweizeitiger Sinuslift).

Ein neueres Verfahren ist der sogenannte crestale Sinuslift, der keine Aufklappung einer Knochenplatte erfordert. Der Zugang zur Kieferhöhle wird vom Kieferkamm her ermöglicht. Dabei wird am zahnlosen Teil des Kieferkamms mittels einer speziell dazu vorgesehenen Stanze (Jesch'sche Stanze) eine Stanzung der Mundschleimhaut bis zum Knochen vorgenommen und mit einer zylindrischen Fräse eine Sackbohrung bis knapp unter die Kieferhöhlenschleimhaut in den Knochen gefräst. Die dabei verwendete Stanze hebt automatisch die Schleimhautscheibe vom Knochen ab und macht eine zentrale Körnung für die weitere Bohrung (Fräsung). Die Bohrung wird im Allgemeinen antral (vom Kamm her) mittels einer Zylinderfräse (beispielsweise mit einem Durchmesser von 3,5 mm) bis ca. 1mm unter den knöchernen Kieferhöhlenboden gefräst, wobei die Knochendicke zuvor mittels Röntgen gemessen wird. Da die Kieferhöhlenschleimhaut nicht durch die Fräse beschädigt werden darf, darf der Kieferknochen nicht mit dem Fräser ganz durchbohrt werden, sodass am Boden der Sackbohrung eine dünne Knochenplatte verbleibt, an deren Rückseite die Kieferhöhlenschleimhaut anliegt.

Herkömmlicher Weise wird diese dünne Knochenplatte dann mit einem zylindrischen Instrument vorsichtig in Richtung Kieferhöhle gestoßen, sodass sie mit der Kieferhöhlenschleimhaut, die oberhalb der Knochenscheibe an dieser haftet, in Richtung Kieferhöhle hineingepresst wird. Dieses "Durchstoßen" der Knochenscheibe stellt für den Eingriff einen kritischen Punkt dar, da ein zu festes Hineindrücken der Knochenscheibe dazu führt, dass die Kieferhöhlenschleimhaut zeltförmig angehoben und gespannt wird, wodurch sie beschädigt werden könnte. Die Kieferhöhlenschleimhaut wird dann vorsichtig angehoben, wonach das Knochenersatzmaterial über die Bohrung in den neu geschaffenen Freiraum eingebracht wird. Das Implantat wird danach meist direkt in der Bohrung verankert.

Auch wenn bereits sehr fortschrittliche Verfahren entwickelt worden sind, um die Kieferhöhlenschleimhaut durch die kleine Bohrung (die meist einen Durchmesser von nur etwa 4mm aufweist) hindurch möglichst schonend und ausreichend weit vom Kieferknochen zu lösen, bleibt der Augenblick, in dem der Kieferknochen durchstoßen wird, ein kritischer Moment, der vom Arzt eine große Erfahrung und ein besonderes Geschick erfordert, wobei trotz aller Umsicht ein Restrisiko besteht, die Kieferhöhlenschleimhaut beim Durchstoßen der Knochenplatte dennoch zu beschädigen.

EP 1269933 beschreibt eine Einrichtung gemäß Oberbegriff des Anspruchs 1.

Um den Sinuslift-Eingriff sicherer zu machen, wären Hilfsmittel wünschenswert, die dieses durchdringende Verlängern der Kieferknochenbohrung erleichtern könnten, und dabei das Risiko, die zarte Sinushaut hinter dem Kieferknochen zu beschädigen, verringern.

Zu diesem Zweck weist die erfindungsgemäße Einrichtung einen Rohrkörper mit einer distalen Arbeitsöffnung und einem der Arbeitsöffnung gegenüberliegenden Eingang auf, der mit einem von einem Schaft eines Arbeitswerkzeugs, z.B. eines Fräsers, durchsetzten, zumindest eine Vorschub- Antriebs- und Steuerbewegung des Fräsers ermöglichenden Dichtungselement verschlossen ist, wobei der Rohrkörper mit einem Anschluss zum Aufbringen eines Innendruckes versehen ist. Der Rohrkörper wird in die zuvor in den Kieferknochen eingebrachte Sackbohrung eingesetzt, wobei die distale Arbeitsöffnung am Ende der Sackbohrung ansteht, wodurch der Innenraum weitgehend dicht abgeschlossen ist. Das im Innenraum des Rohrkörpers befindliche Arbeitsmedium, vorzugsweise NaCl-Lösung, kann nun über den Anschluss unter Druck gesetzt werden, beispielsweise mittels einer mit dem Anschluss verbundenen Spritze. Mit dem Arbeitswerkzeug, das von Außen gesteuert werden kann, wird nun die zwischen Sackbohrung und Kieferhöhle verbleibende Knochenscheibe im Bereich der Arbeitsöffnung abgefräst. In dem Moment, in dem der Kopf des Arbeitswerkzeugs den Knochen durchdringt und in den Bereich unterhalb der Sinushaut eindringt, bewirkt der Überdruck im Innenraum des Rohrkörpers, dass das Arbeitsmedium durch die freie Öffnung dringt, und die dahinterliegende Sinushaut vom Knochen weg, und somit aus dem Arbeitsbereich des Arbeitswerkzeugs drückt. Das Ausströmen des Druckmediums bewirkt einen Druckabfall, der das Durchdringen des Knochens anzeigt und auch ein übermäßiges Aufblähen der Sinushaut verhindert.

In einer vorteilhaften Ausgestaltung der Erfindung kann ein auf dem Rohrkörper in Längsrichtung verstellbarer Vorschubanschlag mit einer Ansatzfläche für ein den Fräser antreibendes Winkelstück vorgesehen sein. Der Vorschubanschlag kann als Stellmutter ausgebildet sein, die eine Ansatzfläche für das Winkelstück aufweist und auf ein im Bereich des Eingangs am Rohrkörper vorgesehenes Gewinde aufschraubbar ist. Ein Vorschubanschlag begrenzt die maximale Eindringtiefe des Arbeitswerkzeugs und erleichtert somit die Handhabung der Einrichtung, wobei ein Verstellen des Anschlags mittels einer Stellmutter eine besonders vorteilhafte Ausführungsform darstellt, da mit der Stellmutter der Vortrieb, mit dem die Fräsung in Richtung Kieferhöhlenschleimhaut durchgeführt wird, einfach und genau geregelt werden kann. Dabei wird die Schonung der Kieferhöhlenschleimhaut durch den Umstand gewährleistet, dass der Vortrieb der Fräse äußerst langsam ist (z.B. nur etwa 1mm/min), und dass schon bei der kleinsten Knochenperforation die Kieferhöhlenschleimhaut sich durch den Druck des Arbeitsmediums aufbläht und von der sie verletzen könnenden Fräse weggedrückt wird. Nachdem sich die Kieferhöhlenschleimhaut aufgebläht hat ist es möglich, etwas weiter zu fräsen um die Knochenperforation noch zu vergrößern.

Wenn das Arbeitswerkzeug ein rotierend angetriebenes Arbeitswerkzeug ist, kann in einer weiteren vorteilhaften Ausgestaltung der Erfindung zwischen einer Eintrittsöffnung der Stellmutter und dem Schaft des Arbeitswerkzeugs ein Rotationskräfte übertragendes Gleitelement vorgesehen sein. Die Rotation der Stellmutter steht im Verhältnis zur Rotation des Schafts, wobei das Gleitelement ein Durchdrehen des Schaftes ermöglicht. Somit wird die Stellmutter durch den schnell rotierenden Schaft während des Fräsens langsam eingeschraubt, sodass der Vorschub des Fräskopfes automatisch verstellt wird.

In vorteilhafter Weise kann an der Stellmutter ein Bremselement angreifen, dessen Anpressdruck über ein Stellorgan regelbar ist. Damit lässt sich die Vorschubgeschwindigkeit der Stellmutter auf einfache Art regeln. Durch festes Andrücken des Bremselements kann die Stellmutter auch fixiert werden, damit ein Vorschub verhindert wird.

In einer weiteren bevorzugten Ausgestaltung der Erfindung kann das Stellorgan in einer Griffverlängerung integriert sein, welche quer oder im Wesentlichen quer zur Achse der Stellmutter von der Einrichtung absteht. Durch die Griffverlängerung lässt sich das Instrument leichter handhaben, wobei vor allem der Anpressdruck des Bremselements und damit die Vorschubgeschwindigkeit, beispielsweise durch eine Stellschraube, einfach und vorzugsweise einhändig regelbar ist.

Zum abdichtenden Einsetzen in die Knochenbohrung kann der in die Sackbohrung einzubringenden Bereich des Rohrkörpers konisch ausgebildet sein. Der konische Bereich kann mit manueller Kraft in die Sackbohrung gepresst werden. Dies ist insbesondere dann vorteilhaft, wenn eine Griffverlängerung vorgesehen ist, die ein Drehen des in die Bohrung eingesetzten Instruments im Mundbereich behindert.

Das Drehen des Instruments kann dazu dienen, das Instrument in die Bohrung einzuschrauben. Zu diesem Zweck kann In einer weiteren Ausführungsform am Rohrkörper in dem in die Sackbohrung einzubringenden Bereich ein selbstschneidendes Außengewinde vorgesehen sein. Dieses sorgt für einen sicheren Halt des Rohrkörpers in der Bohrung und verbessert die Abdichtung gegenüber der Innenwandung der Bohrung. Um diese Abdichtung weiter zu verbessern, kann in vorteilhafter Weise an der Außenseite des Rohrkörpers ein entlang des Rohrkörpers verstellbarer und an diesem lösbar festlegbarer Flansch vorgesehen sein, der einen konischen, zwischen Rohrkörper und Sackbohrungswandung reichenden Dichtungsansatz aufweist. Dieser Flansch wird mit dem Konus gegen die Mundschleimhaut gepresst und dann, z.B. mittels einer Inbusschraube, fixiert.

Zur Erleichterung der Handhabung kann in einer Ausgestaltung der Erfindung am Rohrkörper ein verbreiterter Griffbereich vorgesehen sein.

In einer weiteren vorteilhaften Ausgestaltung der Erfindung kann die Fräseinrichtung über den Anschluss mit einer manuellen oder automatischen Drucksteuereinheit verbunden sein. Diese ermöglicht ein exaktes Steuern und Kontrollieren des Innendrucks, wobei das Durchstoßen des Knochens sofort anhand des Druckabfalls erkennbar ist.

Das Dichtungselement kann in vorteilhafter Weise durch eine oder mehrere O-Ring-Dichtungen gebildet sein. Im Allgemeinen sind an das Dichtungselement keine sehr hohen Anforderungen gestellt, da geringe Undichtheiten unkritisch sind, und beim Dichtungselement austretendes Arbeitsmedium leicht durch die Drucksteuereinheit wieder ausgeglichen werden kann. O-Ring-Dichtungen können daher für eine zufriedenstellende Wirkung ausreichend sein, und stellen somit eine besonders kostengünstige und einfache Ausbildung dar. Zusätzlich dient die O-Ring-Dichtung für den Schaft des Arbeitswerkzeugs als den Schaft zentrierendes, abdichtendes Lager, das nicht nur eine Vorschubbewegung, sondern auch eine taumelnde Steuerbewegung des Arbeitswerkzeugs erlaubt, ohne dass die abdichtenden Eigenschaften wesentlich beeinträchtigt werden.

In einer anderen erfindungsgemäßen Ausgestaltung kann das Dichtungselement durch eine Lochmembran gebildet sein, mit der sich ähnliche Vorteile erzielen lassen, wie mit den oben genannten O-Ring-Dichtungen, wobei jedoch ein größerer Spielraum bei der Dimensionierung der Durchgangsöffnung für das Arbeitswerkzeug möglich ist.

Gemäß der Erfindung können weiters in vorteilhafter Weise an der Stirnfläche des Rohrkörpers über die Arbeitsöffnung vorstehende Stirnhaken vorgesehen sein. Die Stirnhaken dienen, zusätzlich zu dem Schneidgewinde, dem sicheren Halt des Rohrkörpers in der Sackbohrung. Gegenüber dem Schneidegewinde, das in erster Linie in der weichen Spongiosa des Kieferknochens eingeschraubt ist, haben die Stirnhaken den Vorteil, dass sie im Randbereich der am Ende der Sackbohrung verbliebenen Knochenscheibe in die Compacta des Kieferknochens eingreifen.

Bei erfindungsgemäßen Ausführungsformen, bei denen ein herkömmlicher rotierend angetriebener Fräser verwendet wird, erfüllt das Dichtungselement die Funktion einer Wellendichtung und muss den rotierenden Schaft abdichten, ohne dessen Bewegungsfreiheit zu stark einzuschränken. Dies stellt verhältnismäßig hohe Anforderungen an die Qualität des Dichtungselements und führt zu einem raschen Verschleiß desselben. Auch kann der Fräserkopf im Bereich der Arbeitsöffnung die Innenwandung des Rohrkörpers berühren, was zu einer beschleunigten Abnutzung des Fräsers einerseits und andererseits zu einem erhöhten Wärmeaufkommen führt. Weiters können sich bei der Abnutzung des Fräsers Metallspäne lösen, die dann im Operationsbereich verbleiben. Um dies zu vermeiden, kann, in einer weiteren vorteilhaften Ausgestaltung der gegenständlichen Erfindung, das Arbeitswerkzeug ein rotationsfreies Arbeitswerkzeug sein. Rotationsfrei bedeutet im Zusammenhang mit dieser Erfindung, dass das Arbeitswerkzeug und das Dichtungselement im Wesentlichen ohne auftretende Relativgeschwindigkeiten aneinander anliegen, da das Arbeitswerkzeug nicht um seine Hauptachse rotiert. Dadurch entfällt das Erfordernis, einen rotierenden Schaft mittels einer (Rotations-)Wellendichtung abzudichten, was die Anforderungen für das Dichtungselement verringert.

Um die Arbeitsenergie zum abzutragenden Knochen zu übertragen, kann die Einrichtung in vorteilhafter Weise eine Vorrichtung zur Erzeugung bzw. Übertragung mechanischer oder elektromagnetischer Schwingungen aufweisen. Die Relativbewegungen, die bei der Übertragung mechanischer Schwingungen zwischen dem Arbeitswerkzeug und dem Dichtungselement auftreten können, werden durch die Elastizität des Dichtungselements ausgeglichen, sodass bei ausreichend kleiner Schwingungsamplitude die Wirkung des Dichtungselements nicht wesentlich beeinträchtigt ist.

In einer Ausführungsform der Erfindung kann das Arbeitswerkzeug ein piezoelektrisches, chirurgisches Instrument, vorzugsweise ein Ultraschall-Osteotom, sein. Ultraschall-Schneidegeräte für den Medizinischen Einsatz (andere Bezeichnungen dafür sind "Ultraschall-Osteotom" oder "Ultraschall-Knochenfräse") sind im chirurgischen und dentalmedizinischen Fachbereich bekannt und für viele Anwendungen den herkömmlichen, etwa mit einem Winkelstück angetriebenen, rotierenden Instrumenten, in vielerlei Hinsicht überlegen. Im Handel verfügbare Ultraschall-Instrumente bestehen im Wesentlichen aus einem Handstück, in dem der Ultraschallgeber angeordnet ist, und einem auf dem Handstück montierten Ansatz, der für die jeweilige Anwendung speziell ausgebildet ist. Als Ultraschallgeber verwenden diese Systeme meist einen piezoelektrischen Schwingungserzeuger.

Die Ansätze weisen eine für den jeweiligen Einsatz angepasste Form auf und können einen diamantierten Bereich aufweisen, der die Schneideigenschaften des Ansatzes beeinflusst. Über ein Steuergerät können die wesentlichen Einstellungen, dies sind insbesondere die Schwingungsstärke und die Frequenz (gegebenenfalls für mehrere Schwingungsrichtungen, z.B. Horizontal und Vertikal), geregelt werden. Zusätzlich können im Handstück Sensoren vorgesehen sein, etwa zur Messung der elektrischen Resonanz im Handstück, die eine komplexere Steuerung der Betriebsparameter oder auch das Erkennen von Veränderungen der Knochenstruktur erlauben.

Die Verwendung von Ultraschall-Osteotomen hat den Vorteil, dass das Instrument nur auf hartes Gewebe (Knochen) eine schneidende bzw. fräsende Wirkung ausübt und weiches Gewebe (wie zum Beispiel die Sinushaut) nicht beschädigt. Dies gewährleistet eine hohe Präzision und Sicherheit bei geringster Gewebeschädigung. Hinsichtlich der vorliegenden Erfindung ist es zusätzlich von Vorteil, dass der Schaft eines Ultraschallansatzes nicht rotiert, wodurch dieser am Eingang des Rohrkörpers leichter abgedichtet werden kann.

Der mit dem Ultraschall-Osteotom auf den Knochen ausgeübte Druck ist nur gering, sodass ein unbeabsichtigtes Durchstoßen des Knochens, das eine Beschädigung der Sinushaut bewirken könnte, vermieden wird. Da der Anpressdruck bei der Arbeit mit dem Ultraschall-Osteotom deutlich unter dem liegt, der für rotierende Fräser erforderlich ist, ist es leichter möglich, den Fräsvorgang auch "frei", d.h. ohne einen Anschlag, der die Eindringtiefe beschränkt, durchzuführen. Im Gegensatz zum rotierenden Fräskopf, der die Sinushaut bei Kontakt schnell beschädigen kann, ist es mit dem Ultraschall-Osteotom durchaus möglich, die Sinushaut zu berühren und sogar Druck auf diese auszuüben, ohne dass dabei die Sinushaut zwangsläufig beschädigt wird.

In einer bevorzugten Ausführungsform kann das Arbeitswerkzeug ein im Mikrometerbereich, insbesondere in einem Bereich zwischen 20 und 200 Mikrometer, arbeitendes Ultraschall-Osteotom sein. Dabei können auch Einstellungen für mehrere unterschiedliche, sich überlagernde Schwingungen vorgesehen sein, die der Arzt auf die jeweiligen Verhältnisse, insbesondere den Zustand des zu bearbeitenden Knochens und die verwendete Ultraschallspitze, einstellen kann. Beispielsweise kann die effektive Arbeitsschwingung des Handstückes sich aus einer Überlagerung einer stärkeren horizontalen (60 - 200 µm) und einer geringeren vertikalen (20-60 µm) Schwingung ergeben. Die vom Arzt gewählte Einstellung stellt sicher, dass der Knochen während des Schneidens sauber und gekühlt bleibt. Eine Überhitzung des Gewebes kann dadurch verhindert werden.

In einer alternativen Ausgestaltung der Erfindung kann das Arbeitswerkzeug ein Laserschneidegerät sein, welches in vorteilhafter Weise einen vorzugsweise gepulsten CO₂-Laser oder Festkörperlaser aufweisen kann. Laserschneidegeräte für den chirurgischen Einsatz erlauben ein kontaktloses Arbeiten mit einer hohen Präzision. Insbesondere kurz gepulste CO₂-Laser haben sich zum Bearbeiten von Knochenmaterial als sehr effektiv erwiesen. Durch Einstellung der Impulslänge kann eine übermäßige Erwärmung des umliegenden Gewebes verhindert werden. Ein weiterer Vorteil von Laserschneidegeräten im chirurgischen Einsatz ist der geringe Blutaustritt, da Blutgefäße durch den Laser verschlossen werden.

In einer weiteren Ausführungsform der vorliegenden Erfindung kann die Wellenlänge des Lasers auf die Absorptionscharakteristik von Knochengewebe abgestimmt sein. Eine genau abgestimmte Wellenlänge bewirkt, dass der am Knochen einfallende Laserstrahl in einer nur einige Mikrometer dicken Schicht an der Oberfläche des Knochens absorbiert wird, sodass der Laser seine Wirkung nur in diesem Bereich entfaltet. Umliegendes Gewebe, das eine andere Absorptionscharakteristik aufweist, kann dadurch geschont werden. Im Bezug auf CO₂-Laser könnte beispielsweise die wichtigste Emissionslinie des Lasers zwischen 9 und 11 µm liegen. In diesem Bereich ist üblicherweise eine optimale Absorption durch den Knochen zu erwarten. Als "wichtigste Emissionslinie" wird im Allgemeinen der Bereich der höchsten Wertspitze im Emissionsspektrum des Lasers bezeichnet.

In einer weiteren Ausführungsform der vorliegenden Erfindung kann der Laser eine verstellbare Fokussierung aufweisen. Die Fokussierung schränkt den wirksamen Bereich des Lasers auf eine einstellbare Tiefe ein. Indem die Fokussierung des Lasers langsam auf tiefere Bereiche eingestellt wird, kann vermieden werden, dass der Laser die Sinushaut unbeabsichtigter Weise "durchschießt", sobald der Knochen abgetragen ist.

Eine weitere vorteilhafte Ausgestaltung der Erfindung sieht vor, dass das Laserschneidegerät mit einem Endoskop gekoppelt sein kann. Die schnell fortschreitende Technik in der Entwicklung der Endoskopie erlaubt bereits die Verwendung von Mikroendoskopen mit einem Durchmesser von 0,5 mm und weniger. Es ist daher möglich, die in das Instrument eingeführte Spitze des Lasers zusätzlich mit einem Endoskop auszustatten, wobei gegebenenfalls auch derselbe Lichtleiter für den Laser und für das Endoskop verwendet werden kann.

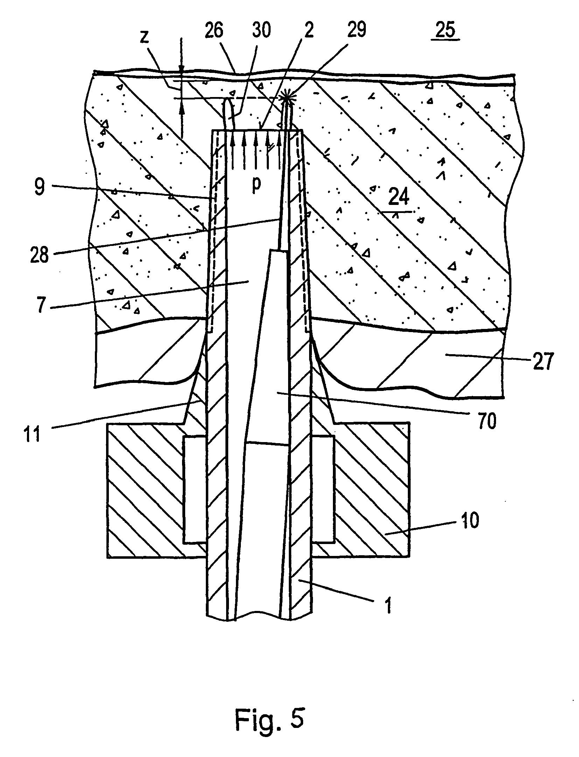

Die Erfindung wird nunmehr anhand der beiliegenden Figuren detailliert beschrieben. Fig. 1 zeigt die erfindungsgemäße Einrichtung im Querschnitt, Fig. 2 ist eine Seitenansicht der Einrichtung, Fig. 3 zeigt in schaubildlicher Darstellung die Spitze der Einrichtung mit davon vorstehenden Stirnhaken, Fig. 4 ist eine Schnittansicht der in einen Kieferknochen eingesetzten erfindungsgemäßen Einrichtung, Fig. 5 veranschaulicht die Verwendung eines Laser-Osteotoms, Fig. 6 zeigt eine Einrichtung mit einer automatisch angetriebenen Stellmutter, Fig. 7 zeigt die Einrichtung mit einer daran montierten Griffverlängerung in einer Seitenansicht und Fig. 8 zeigt die gleiche Einrichtung in einer Draufsicht.

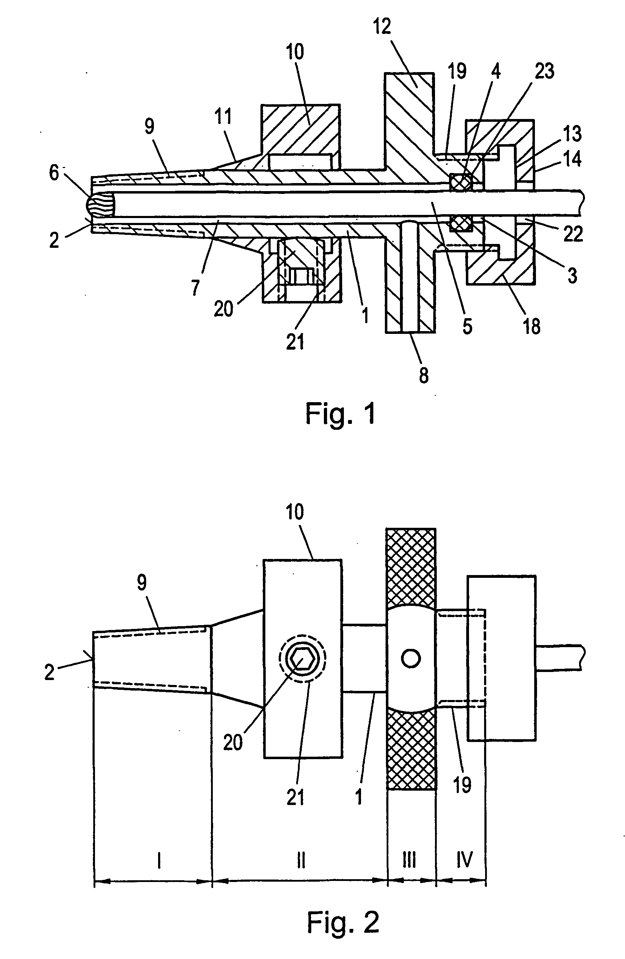

Die in Fig. 1 in einem Querschnitt dargestellte erfindungsgemäße Einrichtung, die in Fig. 2 nochmals in einer Seitenansicht dargestellt ist, besteht aus einem Rohrkörper 1, dessen konisch ausgebildeter Spitzenbereich an der Außenseite des Rohrkörpers 1 mit einem selbstschneidenden Außengewinde 9 versehen ist, das dazu dient, den Rohrkörper 1 in eine im Kieferknochen vorgesehene Sackbohrung einzuschrauben. Der Hohlraum des Rohrkörpers ist im Wesentlichen zylindrisch ausgebildet und erstreckt sich von einer Arbeitsöffnung 2 an der Spitze des Rohrkörpers 1 bis zu einem gegenüber der Arbeitsöffnung 2 liegenden Eingang 3.

Zwischen der Arbeitöffnung 2 und dem Eingang 3 kann der Rohrkörper im Wesentlichen in vier Bereiche eingeteilt werden: Den zuvor beschriebenen konischen Spitzenbereich I mit dem selbstschneidenden Außengewinde 9, einen mittleren zylindrischen Bereich II, auf dem ein Flansch 10 aufgeschoben ist, einen als Haltescheibe 12 ausgebildeten Griffbereich III und einem Endbereich IV, der mit einem Außengewinde versehen ist.

Der auf den zylindrischen Bereich II aufgeschobene Flansch 10 weist an seiner dem Spitzenbereich zugewandten Seite einen konischen Dichtungsansatz 11 auf. In den Flansch 10, der entlang des gesamten zylindrischen Bereichs II verschoben werden kann, ist quer zu seiner Achse eine Gewindebohrung 21 eingebracht, in der eine Imbusschraube 20 eingeschraubt ist. Wenn die Inbusschraube 20 angezogen wird, drückt sie auf den Rohrkörper 1, wodurch der Flansch 10 in seiner Lage fixiert werden kann.

Der Griffbereich III ist im Wesentlichen als flache Haltescheibe 12 ausgebildet, die an ihrer Umfangsfläche vorzugsweise mit einer Rändelung versehen ist, um das Ergreifen und Ein- bzw. Ausschrauben des kleinen Instruments zu erleichtern. Die Haltescheibe 12 weist eine dünne Bohrung auf, die von der Seite her bis zum Hohlraum des Rohrkörpers 1 durchgebohrt ist und die einen Anschluss 8 darstellt. An diesen Anschluss 8 kann ein zu einer Spritze oder einer manuellen oder automatischen Drucksteuereinheit führender Schlauch angebracht werden, über den ein Arbeitsmedium in den Hohlraum des Rohrkörpers 1 eingebracht werden kann.

Auf das Außengewinde 19 des Endbereichs IV ist eine Stellmutter 18 aufgeschraubt, die an ihrer dem Rohrkörper 1 abgewandten Seite einen Vorschubanschlag 13 mit einer Ansatzfläche 14 aufweist. Die Ansatzfläche 14 kann durch Drehen der Stellmutter 18 verstellt werden. Um eine sensible Verstellung der Ansatzfläche zu ermöglichen, ist das Außengewinde 19 vorzugsweise als Feingewinde ausgebildet. In der Mitte der Ansatzfläche 14 weist der Vorschubanschlag 13 eine auf den Hohlraum des Rohrkörpers 1 ausgerichtete Eintrittsöffnung 22 auf, durch die ein Schaft 5 eines Fräsers 5, 6 in den Hohlraum des Rohrkörpers 1 eingeführt ist. Der Durchmesser der Eintrittsöffnung 22 ist etwas größer als der des Schaftes 5 des Fräsers 5, 6, sodass der Fräser 5, 6 im Inneren des Rohrkörpers 1 bewegbar ist.

Um im Inneren des Rohrkörpers 1 eine Druckkammer 7 ausbilden zu können, ist es erforderlich, den Hohlraum des Rohrkörpers 1 und den Schaft 5 des Fräsers 5, 6 im Bereich des Eingangs 3 abzudichten. Als Dichtungselement 4 ist in der in Fig. 1 dargestellten Ausführungsform eine einfache O-Ring-Dichtung vorgesehen, die in einer im Inneren des zylindrischen Hohlraums des Rohrkörpers vorgesehenen Ringnut 23 angeordnet ist. Da eine absolute Dichtheit der Druckkammer 7 nicht unbedingt erforderlich ist, wie im Folgenden noch genauer erörtert werden wird, reicht diese besonders einfache Dichtungsanordnung im Allgemeinen für die Gewährleistung der Funktionalität der Einrichtung aus. Es können jedoch auch nach Belieben andere Dichtungsanordnungen verwenden werden, die im Stand der Technik bekannt sind.

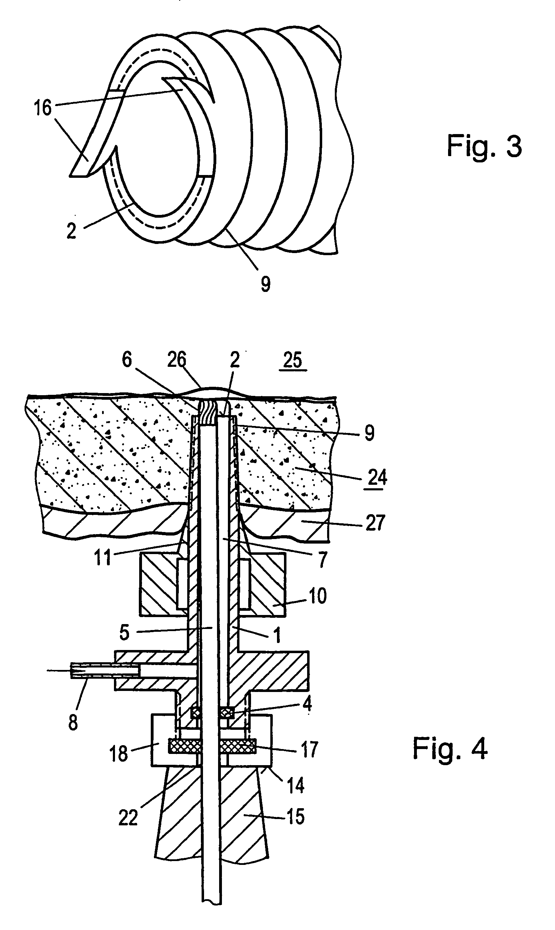

In einer bevorzugten Ausführungsform der Erfindung können an der Spitze des Rohrkörpers 1 Stirnhaken 16 vorgesehen sein, wie sie in Fig. 3 dargestellt sind. Die in Fig. 3 gezeigten Stirnhaken 16 entsprechen im Wensentlichen einer "Verlängerung" bzw. "Ausläufern" der Gewindestufen bzw. Schneiden des selbstschneidenden Außengewindes 9, wobei die Stirnhaken 16 am Rand der Arbeitsöffnung 2 über das Ende des Rohrkörpers 1 vorstehen. Wird nun der Rohrkörper 1 mit dem selbstschneidenden Außengewinde 9 in die zuvor vorbereitete Sackbohrung eingeschraubt, bohren sich die Stirnhaken 16 in die hinter dem Sackloch verbleibende Knochenplatte und sorgen somit für einen festen Halt der Einrichtung. Dies ist insbesondere Vorteilhaft, da das Knochengewebe im Randbereich eines Knochens (der sogenannten Substantia compacta) fester ausgebildet ist als im zentralen Knochenbereich (der Spongiosa), und die Stirnhaken 16 genau in diesem Randbereich eingreifen.

Die Verwendung der erfindungsgemäßen Einrichtung wird nunmehr insbesondere mit Bezugnahme auf Fig. 4 beschreiben, wobei Fig. 4 die erfindungsgemäße Einrichtung bei einem Sinuslift in dem Moment darstellt, in dem der Fräskopf 6 des Fräsers 5, 6 den Kieferknochen durchdringt.

Wie beim herkömmlichen crestalen Sinuslift wird zuvor vom Kieferkamm her eine Sackbohrung in den Kieferknochen 24 eingebracht, wobei eine etwa 1mm tiefe Knochenplatte zwischen dem Ende der Sackbohrung und der Kieferhöhle 25 verbleibt. Dies ist notwendig, um die in der Kieferhöhle 25 am Kieferknochen 24 anliegende Kieferhöhlenschleimhaut 26 nicht zu beschädigen. In die vorbereitete Sackbohrung wird dann der Rohrkörper 1 mit dem selbstschneidenden Außengewinde 9 eingeschraubt, bis die Arbeitsöffnung 2 an der Knochenplatte ansteht, wobei das selbstschneidende Außengewinde 9 und die Stirnhaken 16 dem Rohrkörper 1 einen festen Halt vermitteln.

Zur Verbesserung der abdichtenden Wirkung wird dann der Flansch 10 auf dem Rohrkörper 1 zum Kieferknochen hin verschoben, sodass der auf dem Flansch 10 angeordnete konische Dichtungsansatz 11 am äußeren Rand der Sackbohrung fest gegen die Mundschleimhaut 27 gedrückt wird, und die Bohrung dadurch abdichtet. Gegebenenfalls kann zusätzlich auch ein Kofferdam verwendet werden.

Danach wird der auf einem Winkelstück 15 eingespannte Fräser 5, 6 durch die Eintrittsöffnung 22 und die Dichtungselemente in den Rohrkörper 1 eingeschoben. Die Länge des Schaftes 5 des eingespannten Fräsers 5, 6 (bzw. die Länge des Rohrkörpers 1) ist so bemessen, dass, wenn das Winkelstück 15 an der Ansatzfläche 14 der Stellmutter 18 ansteht, der Fräskopf 6 des Fräsers 5,6 mit der Spitze mit der Knochenplatte in Eingriff gelangt.

Die in Fig. 4 dargestellte Einrichtung weist, neben der O-Ring-Dichtung im Rohrkörper 1, als zusätzliches Dichtungselement eine Lochmembran 17 auf, die innerhalb der Stellmutter 18 angeordnet ist, und den Schaft 5 des Fräsers 5, 6 an der Eintrittsöffnung 22 abdichtet. Die Druckkammer 7 könnte beispielsweise auch nur durch eine Lochmembran 17 abgedichtet werden, die direkt am Eingang 3 des Holraums des Rohrkörpers 1 angeordnet ist. Es könnten auch mehrere Dichtungselemente hintereinander im Rohrkörper 1 angeordnet sein, sofern deren Elastizität ausreicht, um dem Schaft 5 des Fräsers 5, 6 ein leichtes Verschwenken zu ermöglichen, sodass der Kopf des Fräsers 5, 6 im ganzen Bereich der Arbeitsöffnung 2 bewegbar ist.

Der Hohlraum des Rohrkörpers 1 ist somit an beiden Seiten des Rohrkörpers abgedichtet, sodass er eine Druckkammer 7 ausbildet, die über den Anschluss 8 mit einem Arbeitsmedium beaufschlagt werden kann. Im einfachsten Fall kann eine NaCl-Lösung mittels einer Spritze und einem Schlauch in die Druckkammer gedrückt werden. Die Zufuhr kann jedoch auch über eine elektrische Pumpe erfolgen, wobei der vorherrschende Druck, beispielsweise etwa 0,5 bis 2 bar, gemessen und angezeigt werden kann.

Mit dem Fräser 5, 6 wird nun durch eine kreisende Bewegung des Fräskopfes 6 die verbliebene Knochenplatte langsam abgetragen, wobei der Vorschub des Fräsers 5, 6 über die Stellmutter 18 geregelt wird, wodurch sich auch ein sehr langsamer Vorschub, beispielsweise von 1mm/min, erzielen lässt. Das Arbeitsmedium in der Druckkammer 7 dient dabei gleichzeitig der Abfuhr der beim Fräsen erzeugten Wärme und wirkt am Dichtungselement 4 für den rotierenden Schaft 5 als Schmiermittel. Geringe Mengen an Arbeitsmedium, die an dem rotierenden Schaft 5 trotz des Dichtungselements 4 austreten können, stellen für die Funktionalität der Einrichtung kein Problem dar, da der Druck des Arbeitsmediums in der Druckkammer 7 über den Anschluss 8 aufrechterhalten werden kann. Gute Dichteigenschaften des Dichtungselements 4 sind dennoch vorteilhaft, da der Druckabfall im Moment des Durchdringens der Knochenscheibe bei einer dichten Druckkammer 7 besser erkennbar ist.

Sobald der Fräskopf 6 die Knochenscheibe durchbohrt, strömt das unter Druck stehende Arbeitsmedium durch die gebildete Öffnung, und hebt die hinter der Knochenscheibe liegende Kieferhöhlenschleimhaut 26 blasenförmig an. Dies entspricht der in Fig. 4 dargestellten Lage. Die Kieferhöhlenschleimhaut 26, die es zu bewahren gilt, wird also automatisch aus dem Gefahrenbereich gedrückt, den der schnell rotierende Fräskopf 6 für sie darstellt.

Nach dem Durchfräsen der Knochenscheibe kann mit der Fräse 6 der Rand der Bohrung noch ein wenig weiter bearbeitet werden, da die Kieferhöhlenschleimhaut 26 weiterhin blasenförmig angehoben bleibt. Die Kieferhöhlenschleimhaut 26 kann mit dem Arbeitsmedium auch noch weiter abgelöst werden, indem vorsichtig Arbeitsmedium in die Druckkammer und dadurch in den Hohlraum unter der Kieferhöhlenschleimhaut 26 gedrückt wird. Danach wird das erfindungsgemäße Instrument wieder aus der Bohrung herausgeschraubt. Gegebenenfalls kann die Kieferhöhlenschleimhaut 26 mit beliebigen Verfahren weiter abgelöst und angehoben werden, bevor das Knochenersatzmaterial eingebracht und das Implantat in die Bohrung eingeschraubt wird.

Die Erfindung basiert somit auf der Überlegung, dass während der knöchernen Ersteröffnung der Kieferhöhle in Richtung Kieferhöhlenschleimhaut die Sackbohrung ständig unter einem hydrostatischen Druck steht, der bewirkt, dass die Kieferhöhlenschleimhaut bei der kleinsten ersten Perforation des Knochens sich abhebt und vor der Fräse zurückweicht, bzw. "flüchtet". Wie in weiterer Folge beschrieben wird, kann dabei die knöcherne Ersteröffnung nicht nur durch eine Fräse, sondern beispielsweise auch durch andere chirurgische Instrumente, wie etwa einen Bohrer, ein Ultraschallpiezoton oder einen (CO₂-) Laser erfolgen.

Fig. 2 zeigt die Spitze einer weiteren Ausgestaltung der vorliegenden Erfindung. Anstatt der mechanischen Abtragung des Knochenmaterials wird der Kieferknochen 24 mittels eines Laser-Osteotoms 70 bearbeitet. Der in der Fig. 2 dargestellte Teil des Lasers kann beispielsweise das Ende eines Lichtleiters sein, über den der Laserstrahl in das Instrument eingeleitet wird. Der Lichtleiter ist so dünn ausgebildet, dass er in die Druckkammer 7 des Rohrkörpers 1 eingeschoben werden kann. Die Abdichtung erfolgt analog zu anderen beschriebenen Ausführungsformen.

Durch die konische Form der Spitze des Lichtleiters kann der Laserstrahl 28 zielgenau zum Rand der Arbeitsöffnung 2 gelenkt werden, indem der Lichtleiter an die Innenwandung des Rohrkörpers 1 angelegt wird. Durch kreisende Bewegungen mit dem Laser-Osteotom 70 fräst die Bahn des Punktes 29, an dem der Laserstrahl 28 auf den Knochen auftrifft, eine ringförmige Nut 30 in den Knochen.

Mittels eines fokussierten Lasers kann die Entfernung des Wirkpunktes des Laserstrahls von der Spitze des Laser-Osteotoms 70 genau festgelegt werden. Der Fräsvorgang kann entweder durch Verstellen der Fokussierung oder durch langsames einschieben des Laser-Osteotoms 70 genau reguliert werden. Aufgrund der genau definierten Arbeitstiefe des Lasers ist es möglich, die Ringnut 30 bis knapp vor die Sinushaut 26 zu erweitern, ohne diese zu beschädigen.

Sobald der Abstand z zwischen der Sinushaut 26 und dem Ende der Ringnut 30 eine bestimmte Dicke unterschreitet, bricht der Kieferknochen 24 aufgrund des Innendrucks p in der Druckkammer 7, der höher ist als der in der Kieferhöhle 25, entlang der Ringnut 30 durch, noch bevor die Eindringtiefe des Lasers die Sinushaut 26 erreicht hat. Die "kritische" Dicke z ist dabei abhängig von der Knochenbeschaffenheit, der Fläche der Arbeitsöffnung und dem Druck p in der Druckkammer.

Das Durchbrechen der Knochenplatte kann durch den dabei auftretenden Druckabfall in der Druckkammer festgestellt werden, wobei gegebenenfalls der Druckabfall eine automatische Abschaltung des Lasers auslösen kann.

Gegebenenfalls kann das Laser-Osteotom 70 mit einem Mini-Endoskop gekoppelt sein, sodass der Fortschritt der Operation und insbesondere das Durchbrechen der Knochenplatte optisch überwacht werden kann.

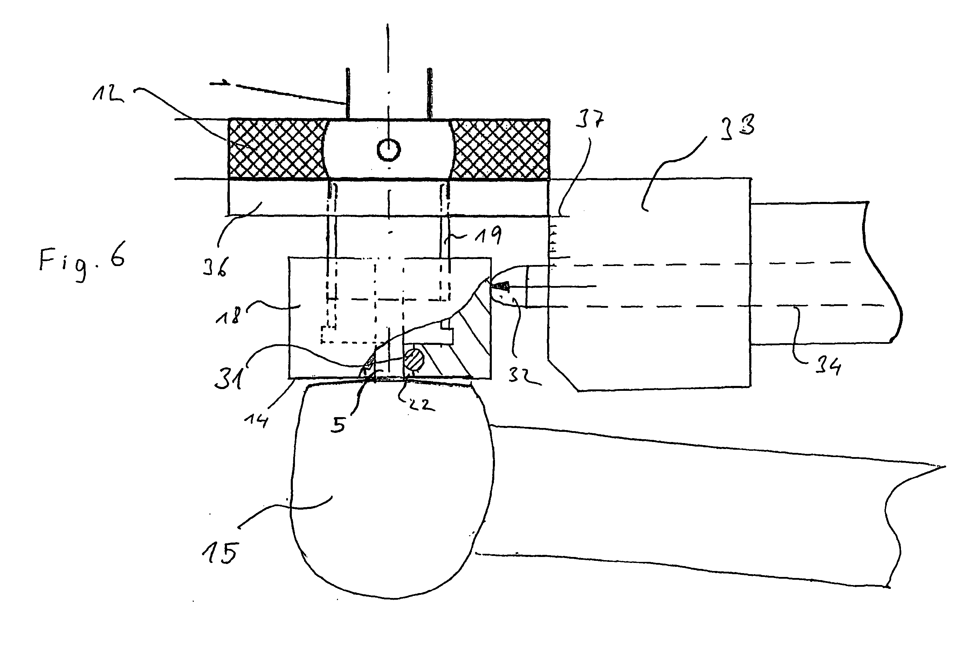

In Fig. 6 ist ein weiteres vorteilhaftes Detail der Erfindung dargestellt, welches eine leicht steuerbare Regelung des Vorschubs für den Fräser ermöglicht. An dem Instrument ist im Bereich der Haltescheibe 12 der Befestigungsansatz 36 einer Griffverlängerung 33 angebracht. Der Befestigungsansatz 36 ist auf das Außengewinde 19 des Endbereichs IV des Rohrkörpers 1 aufgeschraubt und liegt an der Haltescheibe 12 an. Die Befestigung der Griffverlängerung 33, bzw. des Befestigungsansatzes 36 könnte jedoch auch auf andere Weise erfolgen, beispielsweise durch eine Klemmanordnung, gegebenenfalls könnte die Griffverlängerung auch einteilig mit dem Instrument ausgebildet sein.

Im Inneren der Stellmutter 18 ist in der Eintrittsöffnung 22 ein Gleitelement 31 angeordnet, welches zwischen dem rotierenden Schaft 5 und der Stellmutter 18 eine Gleitreibung bewirkt. Das Gleitelement kann vorzugsweise ein einfacher O-Ring sein, der nur einen geringen Druck auf den Schaft 5 ausübt, sodass dieser mit hohem Schlupf in dem O-Ring rotiert und nur eine geringe Rotationsenergie auf die Stellmutter 18 überträgt. Die Gleitreibung treibt die Stellmutter 18 an, die dadurch langsam über das Außengewinde 19 eingeschraubt wird, sodass die Ansatzfläche 14 und somit der Vorschub des Fräskopfes ebenfalls bewegt wird. Gegebenenfalls kann das Gleitelement 31 das Dichtungselement 4 im Rohrkörper ersetzen.

An der Stellmutter 18 greift ein Bremselement 32 an, welches über eine Bremsstange 34 in Pfeilrichtung gegen die zylinderförmig ausgebildete Außenfläche der Stellmutter 18 drückt. Durch Verstellen des Anpressdrucks kann die Rotation der Stellmutter 18 stärker oder schwächer gebremst oder gestoppt werden.

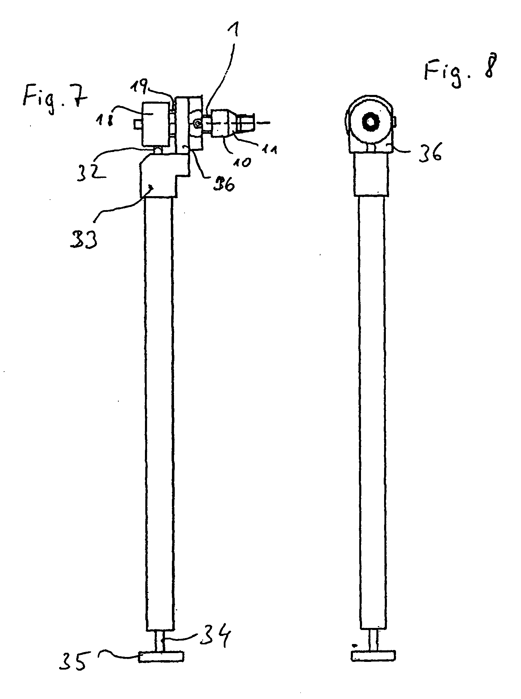

Die Griffverlängerung 33 ist nochmals in Fig. 7 und 8 vollständig dargestellt, wobei an dem dem Bremselement 32 gegenüberliegenden Ende der Bremsstange 34 ein leicht zugängliches Stellrad 35 angeordnet ist, mit dem die Bremsstange über ein Gewinde (nicht dargestellt) im Inneren der Griffverlängerung ein- und ausgeschraubt werden kann. Das Stellrad 35 kann vom Operateur einhändig bedient werden, während die andere Hand das Winkelstück bedient.

Am Anfang des Fräsvorgangs ist die Bremse fest angezogen, sodass die Anschlagfläche 14 einen zuvor festgelegten Abstand von der Arbeitsöffnung 2 aufweist, sodass der Fräskopf 6 nicht tiefer als vorgesehen in den Kieferknochen eindringen kann. Um mit dem Fräskopf tiefer in Richtung Kieferhöhle vorzudringen, löst der Operateur durch Drehen des Stellrads 35 die Bremse, sodass die Stellmutter 18 ein Stück weit mit dem Fräserschaft 5 mitgedreht und eingeschraubt wird, woraufhin die Bremse wieder festgezogen wird. Durch Beobachten der Stellmutter kann der Arzt den eingestellten Vorschub kontrollieren, wobei eine Skala 37 das Abschätzen der Eindringtiefe erleichtern kann. Die Skala kann entweder auf einem Teil der Griffverlängerung oder auch an einer anderen Stelle des Instruments angeordnet sein.

## Patentansprüche

1. Einrichtung zum durchdringenden Verlängern einer in hartes Gewebe, insbesondere den Kieferknochen, eingebrachten Sackbohrung, wobei die Einrichtung einen in die Knochenbohrung einsetzbaren Rohrkörper (1) aufweist, wobei der Rohrkörper (1) eine distale Arbeitsöffnung (2) und einen der Arbeitsöffnung (2) gegenüberliegenden Eingang (3) aufweist, der mit einem von einem Schaft (5) eines Werkzeugs durchsetzten, zumindest eine Vorschub-, Antriebs- und Steuerbewegung des Arbeitswerkzeugs (5,6) ermöglichenden Dichtungselement (4) verschlossen ist, und wobei der Rohrkörper (1) mit einem Anschluss (8) zum Aufbringen eines Innendruckes versehen ist, **dadurch gekennzeichnet, dass** der Rohrkörper (1) so ausgestaltet ist, dass er in die Knochenbohrung dicht einsetzbar ist, oder dass am Rohrkörper (1) Mittel zum dichten Einsetzen in die Knochenbohrung vorgesehen sind; und dass das Werkzeug ein Arbeitswerkzeug, insbesondere ein Fräser (5, 6) ist.

2. Einrichtung nach Anspruch 1, **dadurch gekennzeichnet, dass** ein auf dem Rohrkörper (1) In Längsrichtung verstellbarer Vorschubanschlag (13) mit einer Ansatzfläche (14) für ein das Arbeitswerkzeug (5, 6) antreibendes Winkelstück (15) vorgesehen ist.

3. Einrichtung nach Anspruch 2, **dadurch gekennzeichnet, dass** der Vorschubanschlag (13) als Stellmutter (18) ausgebildet ist, die eine Ansatzfläche (14) für das Winkelstück (15) aufweist und auf ein im Bereich des Eingangs (3) am Rohrkörper (1) vorgesehenes Gewinde (19) aufschraubbar ist.

4. Einrichtung nach Anspruch 3, **dadurch gekennzeichnet, dass** das Arbeitswerkzeug ein rotierend angetriebenes Arbeitswerkzeug ist, und dass zwischen einer Eintrittsöffnung (22) der Stellmutter (18) und dem Schaft (5) des Arbeitswerkzeugs ein Rotationskräfte übertragendes Gleitelement (31) vorgesehen ist.

5. Einrichtung nach Anspruch 4, **dadurch gekennzeichnet, dass** an der Stellmutter (18) ein Bremselement (32) angreift, dessen Anpressdruck über ein Stellorgan regelbar ist.

6. Einrichtung nach Anspruch 5, **dadurch gekennzeichnet, dass** das Stellorgan in einer Griffveriängerung (33) integriert ist, welche quer oder im Wesentlichen quer zur Achse der Stellmutter (18) von der Einrichtung absteht.

7. Einrichtung nach einem der Ansprüche 1 bis 6, **dadurch gekennzeichnet, dass** am Rohrkörper (1) in dem in die Sackbohrung einzubringenden Bereich ein selbstschneidendes Außengewinde (9) vorgesehen ist.

8. Einrichtung nach einem der Ansprüche 1 bis 7, **dadurch gekennzeichnet, dass** an der Außenseite des Rohrkörpers (1) ein entlang des Rohrkörpers (1) verstellbarer und an diesem lösbar festlegbarer Flansch (10) vorgesehen ist, der einen konischen, zwischen Rohrkörper (1) und Sackbohrungswandung reichenden Dichtungsansatz (11) aufweist.

9. Einrichtung nach einem der Ansprüche 1 bis 8, **dadurch gekennzeichnet, dass** am Rohrkörper (1) ein vorzugsweise als Haltescheibe (12) ausgebildeter Griffbereich (III) vorgesehen ist.

10. Einrichtung nach einem der Ansprüche 1 bis 9, **dadurch gekennzeichnet, dass** die Einrichtung über den Anschluss (8) mit einer manuellen oder automatischen Drucksteuereinheit verbunden ist.

11. Einrichtung nach einem der Ansprüche 1 bis 10, **dadurch gekennzeichnet, dass** das Dichtungselement (4) durch eine oder mehrere O-Ring-Dichtungen gebildet ist, oder dass das Dichtungselement (4) durch eine Lochmembran (17) gebildet ist, und/oder dass an der Stirnfläche des Rohrkörpers (1) über die Arbeitsöffnung (2) vorstehende Stirnhaken (16) vorgesehen sind.

12. Einrichtung nach Anspruch 1, **dadurch gekennzeichnet, dass** das Arbeitswerkzeug ein rotationsfreies Arbeitswerkzeug ist.

13. Einrichtung nach Anspruch 12, **dadurch gekennzeichnet, dass** die Einrichtung eine Vorrichtung zur Erzeugung bzw. Übertragung mechanischer oder elektromagnetischer Schwingungen aufweist.

14. Einrichtung nach Anspruch 13, **dadurch gekennzeichnet, dass** das Arbeitswerkzeug ein piezoelektrisches, chirurgisches Instrument, vorzugsweise ein Ultraschall-Osteotom, ist, und dass das Arbeitswerkzeug insbesondere ein im Mikrometerbereich, insbesondere in einem Bereich zwischen 20 und 200 Mikrometer, arbeitendes Ultraschall-Osteotom ist.

15. Einrichtung nach Anspruch 14, **dadurch gekennzeichnet, dass** das Arbeitswerkzeug ein Laserschneidegerät ist,
und/oder dass das Laserschneidegerät einen vorzugsweise gepulsten CO₂-Laser oder Festkörperlaser aufweist.
und/oder dass die Wellenlänge des Lasers auf die Absorptionscharakteristik von Knochengewebe abgestimmt ist.
und/oder dass der Laser eine verstellbare Fokussierung aufweist,
und/oder dass das Laserschneldegerät mit einem Endoskop gekoppelt ist.

## Claims

1. A device for extending a blind hole penetrating hard tissue, particularly the jaw bone, said device having a tubular body (1) insertable into said hole in the bone, said tubular body (1) having a distal work opening (2) and an inlet opening (3), which is located opposite said work opening (2) and closed by at least one sealing element (4), through which the shaft (5) of an instrument extends and which allows for advancing, driving and controlling movements of said instrument (5, 6), and said tubular body (1) being provided with a supply (8) for applying internal pressure, **characterised in that** said tubular body (1) is configured so that it can be inserted into the hole in the bone so that it fits tightly therein or **in that** means for inserting it into the hole in the bone so that it fits tightly therein are provided on the tubular body (1), and **in that** said instrument is a work instrument, particularly a dental mill (5, 6).

2. The device according to claim 1, **characterised in that** an advancement stop (13) which is adjustable in a longitudinal direction and has a fitting surface (14) for an angled part (15) driving said work instrument (5, 6) is provided on the tubular body (1).

3. The device according to claim 2, **characterised in that** said advancement stop (13) is formed as an adjusting nut (18), which has a fitting surface (14) for said angled part (15) and may be screwed onto a thread (19) provided in the area of the inlet opening (3) on the tubular body (1).

4. The device according to claim 3, **characterised in that** said work instrument is a rotatingly driven work instrument and **in that** a sliding element (31) which transmits the rotary forces is provided between an inlet opening (22) of the adjusting nut (18) and the work instrument shaft (5).

5. The device according to claim 4, **characterised in that** a breaking element (32) engages with said adjusting nut (18), the pressure applied by said breaking element (32) being adjustable by means of a control unit.

6. The device according to claim 5, **characterised in that** said control unit is integrated into a handle extension (33) which projects from the device transversely or essentially transversely to the axis of the adjusting nut (18).

7. The device according to any one of the claims 1 to 6, **characterised in that** a self-tapping outside thread (9) is provided on the tubular body (1) in the area which is to be inserted into the blind hole.

8. The device according to any one of the claims 1 to 7, **characterised in that** a flange (10) which can be adjusted along the tubular body (1) and releasably fixed thereto is provided on the outside of said tubular body (1), said flange having a conical sealing neck portion (11) extending between the tubular body (1) and the walls of the blind hole.

9. The device according to any one of the claims 1 to 8, **characterised in that** a handle area (III) is provided on the tubular body (1), preferably in the form of a disc-shaped handle (12).

10. The device according to any one of the claims 1 to 9, **characterised in that** said device is connected to a manual or automatic pressure control unit via a supply (8).

11. The device according to any one of the claims 1 to 10, **characterised in that** said sealing element (4) is formed by one or more O-ring seals or **in that** said sealing element (4) is formed by a perforated membrane (17), and/or **in that** the front face of the tubular body (1) is provided with frontbarbs (16) projecting over the work opening (2).

12. The device according to claim 1, **characterised in that** said work instrument is a non-rotating work instrument.

13. The device according to claim 12, **characterised in that** said device has a means for generating and transmitting mechanical or electromagnetic oscillations.

14. The device according to claim 13, **characterised in that** said work instrument is a piezoelectric surgical instrument, preferably an ultrasonic osteotome, and **in that** said work instrument particularly is an ultrasonic osteotome adapted for the micron range, particularly for the range between 20 and 200 microns.

15. The device according to claim 14, **characterised in that** the work instrument is a laser cutting instrument,
and/or **in that** said laser cutting instrument has a preferably pulsed CO₂ laser or solid-state laser,
and/or **in that** the laser wave length is adjusted to the absorption characteristics of the bone tissue,
and/or **in that** the laser's focussing is adjustable,
and/or **in that** said laser cutting instrument is coupled to an endoscope.

## Revendications

1. Dispositif pour le rallongement d'un perçage borgne pénétrant un tissu dur, notamment l'os maxillaire, ledit dispositif comportant un corps tubulaire (1) insérable dans ledit trou dans l'os, ledit corps tubulaire (1) comportant une ouverture de travail (2) distale et une ouverture d'entrée (3) située à l'opposé de ladite ouverture de travail (2) et fermée par au moins un élément d'étanchéité (4) traversé par une tige (5) d'un instrument, permettant au moins des mouvements d'avancement, de propulsion et de contrôle dudit instrument de travail (5, 6), et ledit corps tubulaire (1) étant doté d'un branchement (8) pour l'application d'une pression interne, **caractérisé en ce que** ledit corps tubulaire (1) est configuré en sorte qu'il puisse être inséré hermétiquement dans le trou de l'os ou **en ce que** des moyens pour son insertion hermétique dans le trou sont prévus sur le corps tubulaire (1), et **en ce que** l'instrument est un instrument de travail, notamment une fraise (5,6).

2. Dispositif selon la revendication 1, **caractérisé en ce qu'**un arrêt d'avance (13) ajustable dans le sens longitudinal avec une surface d'application (14) pour une pièce coudée (15) propulsant l'instrument de travail (5, 6) est prévu sur le corps tubulaire (1).

3. Dispositif selon la revendication 2, **caractérisé en ce que** ledit arrêt d'avance (13) est configuré à la manière d'un écrou de réglage (18) comportant une surface d'application (14) pour la pièce coudée (15) et pouvant étre vissé sur un filet (19) prévu dans la région de l'ouverture d'entrée sur le corps tubulaire (1).

4. Dispositif selon la revendication 3, **caractérisé en ce que** l'outil de travail est un outil de travail propulsé par et **en ce qu'**un élément glisssant (31), transmettant les forces de rotation, est prévu entre une ouverture d'entrée (22) de l'écrou de réglage (18) et la tige (5) de l'instrument de travail.

5. Dispositif selon la revendication 4, **caractérisé en ce que** ledit écrou de réglage (18) est engagé avec un élément de freinage (32), la pression exercée par ledit élément de freinage (32) étant réglable par une unité de réglage.

6. Dispositif selon la revendication 5, **caractérisé en ce que** ladite unité de réglage est intégrée dans une extension (33) d'une manche qui s'étend à partir du dispositif transversalement ou essentiellement transversalement à l'axe de l'écrou de réglage (18).

7. Dispositif selon une des revendications 1 à 6, **caractérisé en ce que** un filet extérieur (9) autotaraudant est prévu sur le corps tubulare (1) dans la région à insérer dans le perçage borgne.

8. Dispositif selon une des revendications 1 à 7, **caractérisé en ce qu'**un bride (10) ajustable le long du corps tubulaire (1) et fixé de manière amovible sur celui-ciest prévu sur l'extérieur dudit corps tubulaire (1), ledit bride (10) comportant un rebord d'étanchéité (11) conique s'étendant entre le corps tubulaire (1) et les parois du perçage borgne.

9. Dispositif selon une des revendications 1 à 8, **caractérisé en ce que** une région de préhension (III), de préférence étant configurée à la manière d'un disque de préhension (12), est prévue sur le corps tubulaire (1).

10. Dispositif selon une des revendications 1 à 9, **caractérisé en ce que** le dispositif est lié par le branchement (8) à une unité de contrôle de pression manuelle ou automatique.

11. Dispositif selon une des revendications 1 à 10, **caractérisé en ce que** l'élément d'étanchéité (4) est formé par un ou plusieurs joints toriques ou **en ce que** l'élément d'étanchéité (4) est formé par une membrane perforée (17) et/ou **en ce que** des barbelures frontales (16) faisant saillie à l'ouverture de travail (2) sont prévues sur la surface frontale du corps tubulaire (1).

12. Dispositif selon la revendication 1, **caractérisé en ce que** l'instrument de travail est un instrument de travail non-tournant.

13. Dispositif selon la revendication 12, **caractérisé en ce que** le dispositif comporte un moyen pour générer et transmettre des oscillations mécaniques ou électromagnétiques.

14. Dispositif selon la revendication 13, **caractérisé en ce que** l'instrument de travail est un instrument piézoélectrique chirurgical, de préférence un ostéotome à ultrasons, et **en ce que** l'instrument de travail est notamment un ostéotome à ultrasons agissant à l'échelle du micron, notamment entre 20 et 200 microns.

15. Dispositif selon la revendication 14, **caractérisé en ce que** l'instrument de travail est un instrument d'incision laser,
et/ou **en ce que** ledit instrument d'incision laser comporte de préférence un laser au CO₂ pulsé ou un laser au solide pulsé,
et/ou **en ce que** la longueur d'onde du laser est adaptée aux caractéristiques d'absorption du tissu osseux,
et/ou **en ce que** le laser comporte une focalisation réglable,
et/ou **en ce que** l'instrument d'incision laser est lié à un endoscope.
